# EUROPÄISCHE PATENTSCHRIFT

(11) **EP 0 741 727 B1**
(45) Veröffentlichungstag und Bekanntmachung des Hinweises auf die Patenterteilung: **28.03.2001**
(21) Anmeldenummer: 95906212.6
(22) Anmeldetag: 31.01.1995
(51) Int. Cl.: C07D 471/20, C07B 55/00

(54) **VERFAHREN ZUR ISOMERISIERUNG VON SPIROZYKLISCHEN BETA-AMINOCARBOXYL- UND BETA-AMINOCARBONYLVERBINDUNGEN**
PROCESS FOR ISOMERIZATION OF SPIROCYCLIC COMPOUNDS WITH BETA-AMINOCARBOXYL AND/OR BETA-AMINOCARBONYL SYSTEMS
PROCEDE D'ISOMERISATION DE COMPOSES SPIROCYCLIQUES COMPORTANT DES SYSTEMES BETA-AMINOCARBOXYLE ET/OU BETA-AMINOCARBONYLE

(30) Priorität: 04.02.1994 AT 21294
(43) Veröffentlichungstag der Anmeldung: 13.11.1996
(73) Patentinhaber: IMMODAL PHARMAKA GESELLSCHAFT m.b.H., 6111 Volders (AT)
(72) Erfinder: KEPLINGER, Dietmar, A-6020 Innsbruck (AT); KEPLINGER, Klaus, A-6020 Innsbruck (AT); LAUS, Gerhard, A-6020 Innsbruck (AT)
(74) Vertreter: Hofinger, Engelbert, Dr.Dr.
(86) Internationale Anmeldenummer: AT9500019
(87) Internationale Veröffentlichungsnummer: WO9521169

(56) Entgegenhaltungen:
- CHEMISTRY OF NATURAL COMPOUNDS, Bd. 12, Nr. 3, 1976 Seiten 355-356, M. R. SHARIPOV ET AL. 'Alkaloids of Vinca erecta'
- CHEMISTRY OF NATURAL COMPOUNDS, Bd. 12, Nr. 2, 1976 Seiten 201-202, M. R. YAGUDAEV ET AL. 'Structure of Herboxine'
- ANALES DE QUIMICA, SER. C, Bd. 78, Nr. 2, 1982 Seiten 180-183, J. BORGES DEL CASTILLO ET AL. 'Flora salvadorena. V. Estudio de los alcaloides de la hamelia patens jaco'
- PHYTOCHEMISTRY, Bd. 14, Nr. 2, 1975 Seiten 565-568, F. TITEUX ET AL. 'Structure des caboxines: Alcaloides oxindoliques du Cabucala fasciculata'
- TETRAHEDRON, Bd. 34, Nr. 22, 1978 Seiten 3341-3344, P. L. MAJUMDER ET AL. 'Stereochemistry and relative stability of the oxindoles derived from venenatine and alstovenine. The two C-3 epimeric 9-methoxy-D/E-cis-yohimbinoid alkaloids of Alstonia venenata R.Br.'
- J. CHROMATOGR., Bd. 662, Nr. 2, 1994 Seiten 243-249, G. LAUS, D. KEPLINGER 'Separation of stereoisomeric oxindole alkaloids from Uncaria tomentosa by high performance liquid chromatography'

## Beschreibung

In der Natur kommen Verbindungen mit spirozyklischem β-Aminocarboxyl-System vor. Es sind dies die Oxindolalkaloide. Verbindungen mit spirozyklischen β-Aminocarbonyl-System sind bisher nicht gefunden worden. Ihre Synthese oder ihre Derivatisierung aus Oxindolalkaloiden ist jedoch denkbar und könnte zum Beispiel zu ihrer Verwendung als Therapeutikum sinnvoll sein.

Beide spirozyklischen Systeme haben gemeinsam, dass sie unter bestimmten Bedingungen Isomerisierung ermöglichen, so dass mit ihnen ausgestattete Verbindungen in zumindest zwei Isomeren vorkommen. Wenn solche Verbindungen auch mit cis-verknüpften Ringsystemen vorliegen, bilden sie zumindest vier Isomeren.

Aus zahlreichen Pflanzen, vor allem der Gattung Uncaria, sind Isomerenfamilien mit zwei Mitgliedern, zum Beispiel Mitraphyllin - Isomitraphyllin, und Isomerenfamilien mit vier Mitgliedern, zum Beispiel Pteropodin - Isopteropodin - Speciophyllin - Uncarin F, isoliert worden (A.F.Phillipson, S.R.Hemingway, C.E.Ridsdale, Lloydia (J.Nat.Prod.), 41 (1978), 503).

An solchen Substanzen ist die Isomerisierung der Oxindolalkaloide erkannt und auch beschrieben worden (A.F.Beecham, N.K.Hart, S.R.Johns, J.A.Lamberton, Aust.J.Chem. 21 (1968), 491; J.-L.Pousset, J.Poisson, R.J.Shine, M.Shamma, Bull.Soc.chim. (1967), 2766). Wesentliches aber, die Isomerisierungsbedingungen vor allem, ist äusserst mangelhaft oder irreführend beschrieben. Ein Vorgehen zur Herstellung definierter Isomerengemische nach den bisher vorliegenden Literaturangaben erwies sich als unmöglich. Umfangreiche eigene Untersuchungen waren daher nötig, um im technischen Massstab die Herstellung definierter Isomerengemische zu ermöglichen.

### Die Untersuchungen:

### 1. Die Lösungsmittel :

Aus Literaturangaben war bekannt, dass Oxindolalkaloide in polaren Lösungsmitteln isomerisieren. Es wurde daher Hexafluor-lsopropanol als hochpolares Lösungsmittel auf seine Eignung als Isomerisierungsmedium untersucht. Dazu wurde Pteropodin in einer Konzentration von 18 mg/l gelöst und über 8 Tage bei 20°C stehen gelassen. Laufend wurden Proben entnommen und auf den Pteropodingehalt untersucht. Eine Isomerisierung wurde nicht festgestellt. Auch wurde in der Literatur als Lösungsmittel zur Isomerisierung Pyridin angegeben, dessen Polarität sehr niedrig ist. Eigene Untersuchungen bestätigten die Richtigkeit dieser Angabe unter der Voraussetzung, dass im Pyridin Reste von Wasser gelöst werden. Es erwies sich also, dass die Polarität eines Lösungsmittels zur Erklärung der Isomerisierung von Oxindolalkaloiden nicht herangezogen werden kann.

Wesentliche Ursachen der Polarität eines Lösungsmittels sind seine Dielektrizitätskonstante und seine Fähigkeit zur Wasserstoff-Brückenbildung. Es wurde daher nach einem Zusammenhang zwischen der Dielektrizitätskonstante und der Reaktionsgeschwindigkeit gesucht (Tab. 1).

**Tab. 1 :**

| *Abhängigkeit der Isomerisierungsgeschwindigkeit von Pteropodin von der Dielektrizitätskonstante* ε *in reinen Lösungsmitteln bei 50° C* ^{(a)} | | |
|---|---|---|
| LM | ε (50° C) | k |
| Formamid | 100 | 0.0051 |
| Wasser | 70 | 0.82 |
| Dimethylsulfoxid | ca. 46 | 0.0011 |
| Glycerin | 38 | 0.0072 |
| Methanol | 27 | 0.0048 |
| Pyridin | 11 | 0.00014 |
| Dioxan | 2 | 0.00008 |

| | | |
|---|---|---|
| ^{(a)} Die angegebene Geschwindigkeitskonstante k wurde aus der Anfangsgeschwindigkeit der Abnahme der Pteropodin-Konzentration bestimmt und bedeutet also die Summe der drei Einzelkonstanten k=k₁₂+k₁₃+k₁₄ für die Umwandlung von Pteropodin in die drei anderen Isomeren, normiert auf eine Gesamtalkaloidkonzentration von 1, und hat die Dimension [h ⁻¹]. Die Konstante k₁₂ beschreibt die Umwandlung von Pteropodin in Isopteropodin, k₁₃ in Speciophyllin und k₁₄ in Uncarin F. | | |

Es wurde festgestellt, dass auch durch die Dielektrizitätskonstante die Isomerisierung nicht befriedigend erklärt werden kann. Es fiel jedoch auf, dass Lösungsmittel mit der Fähigkeit, als Protonendonoren Wasserstoffbrücken auszubilden, wie Formamid, Wasser, Glyzerin oder Methanol, eine schnellere Isomerisierung bewirkten, als solche ohne diese Fähigkeit.

Als Reaktionsmedium zur Isomerisierung geeignet sind nach diesen Untersuchungen polare Lösungsmittel nur dann, wenn sie neben einer relativ hohen Dielektrizitätskonstante als Protonendonoren auch Wasserstoffbrücken ausbilden.

Es wurde daher versucht, Lösungsmittelmischungen aus einem Teil mit der Fähigkeit, als Protonendonor Wasserstoffbrücken auszubilden, und aus einem Teil mit hoher Dielektrizitätskonstante herzustellen. Mischungen von Formamid und Alkoholen erwiesen sich dabei als gute Reaktionmedien (Tab. 2).

**Tab. 2 :**

| *Isomerisierunggeschwindigkeit von Pteropodin in Lösungsmittelgemischen bei 50°C* ^{(a)} | |
|---|---|
| LM | k |
| Formamid-Methanol (1:1) | 0.0050 |
| Formamid-Hexafluorisopropanol (1:1) | 0.0037 |
| Methanol-Dioxan (1:1) | 0.0024 |
| Methanol-Dimethylsulfoxid (1:1) | 0.0018 |

| | |
|---|---|
| ^{(a)} Siehe Tab. 1 | |

Wasser vereinigt als einziges reines Lösungsmittel eine hohe Dielektrizitätskonstante und die Fähigkeit, als Protonendonor Wasserstoffbrücken auszubilden in einem Ausmass in sich, dass befriedigende Reaktionsgeschwindigkeiten erzielt werden. In reinem Wasser sind Oxindolalkaloide jedoch praktisch unlöslich. Es war daher naheliegend, geeignete Lösungsvermittler zur Isomerisierung in Wasser zu suchen.

Bei diesbezüglichen Untersuchungen wurde festgestellt, dass die Art des Lösungsvermittlers erheblichen Einfluss auf die Isomerisierungsgeschwindigkeit ausübt (Tab. 3).

**Tab. 3 :**

| *Einfluss des Lösungsvermittlers auf die Isomerisierungsgeschwindigkeit von Pteropodin in Wasser bei 50° C* ^{(a)} | | |
|---|---|---|
| LM | ε (Lösungsvermittler) | k |
| Wasser-Formamid (1:1) | 100 | 0.067 |
| Wasser-Methanol (1:1) | 27 | 0.043 |
| Wasser-Acetonitril (1:1) | ca. 32 | 0.042 |
| Wasser-Pyridin (1:1) | 11 | 0.0002 |
| Wasser-Dioxan (1:1) | 2 | 0.012 |

| | | |
|---|---|---|
| ^{(a)} Siehe Tab. 1 | | |

Zur Isomerisierung in Wasser können nur Lösungsvermittler mit relativ hoher Dielektrizitätskonstante ab etwa ε = 20 eingesetzt werden, die auch als Protonendonoren Wasserstoffbrücken ausbilden.

Die Abhängigkeit des Lösungsvermögens von der Konzentration eines Lösungsvermittlers wurde wegen seiner guten Eignung am Methanol untersucht.

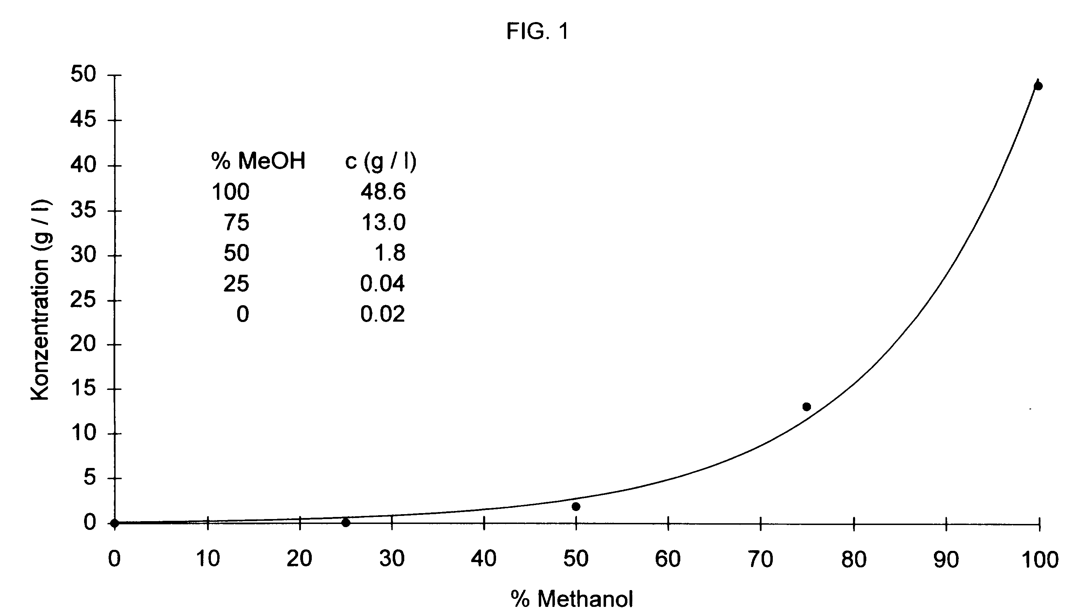

Es zeigte sich, dass das Lösungsvermögen eine exponentielle Funktion der Konzentration des Lösungsvermittlers im Wasser ist (Fig. 1).

### 2. Die Isomerisierung :

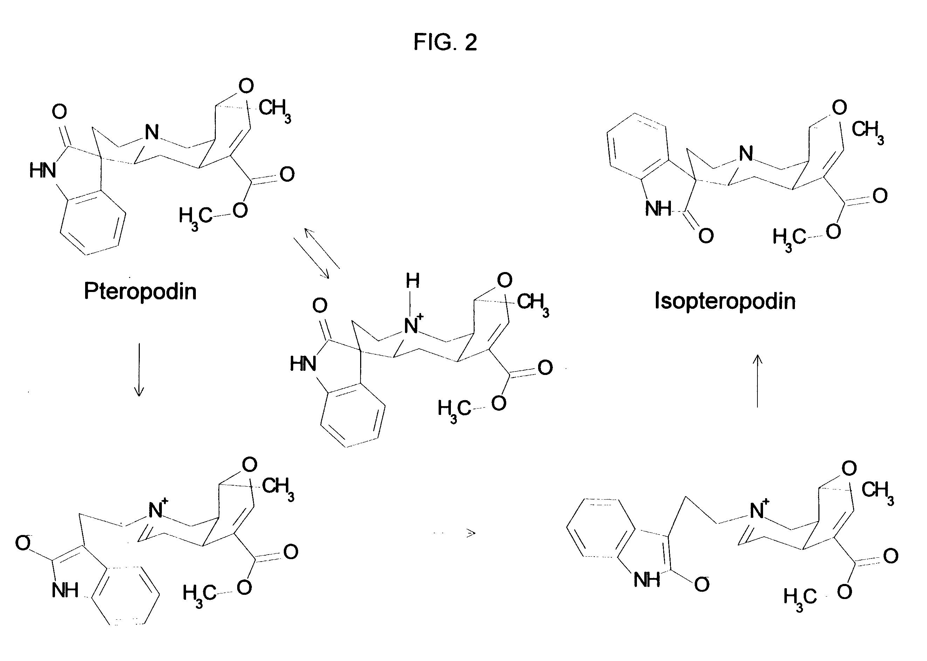

Aus der Literatur (J.-L.Pousset, J.Poisson, R.J.Shine, M.Shamma, Bull.Soc.chim. (1967), 2766) ist bekannt, dass Oxindolalkaloide über eine zwitterionische Zwischenstufe isomerisieren, die sich am β-Aminocarboxyl-System ausbildet. Dabei wird zwischen dem Aminostickstoff und dem β-Kohlenstoff eine zweite Bindung ausgebildet, die Bindung zwischen α- und β-Kohlenstoff wird gelöst und das Spirozentrum am α-Kohlenstoff wird frei zur Inversion (Fig. 2).

Beschrieben wurde auch, dass sich durch Kochen in einer Säure bevorzugt die Form bildet, bei der das Proton am Amino-Stickstoff und der Carboxyl-Sauerstoff in syn-Stellung zueinander stehen, durch Kochen in Pyridin aber das Isomere in anti-Stellung (J.D.Phillipson, S.R.Hemingway, Phytochemistry, 12 (1973), 2795).

Eigene Untersuchungen haben gezeigt, dass unterschiedliche pH-Werte zu unterschiedlichen Isomerengleichgewichten führen.

Ein Lösungsmittel Wasser-Methanol im Verhältnis 9:1 wurde durch Zugabe von Phosphorsäure bzw. Natronlauge auf pH-Werte von 2 bis 9 eingestellt. In diesen Lösungsmitteln wurde Pteropodin in einer Konzentration von 30 mg/l gelöst und bis zur Endzusammensetzung ausisomerisiert.

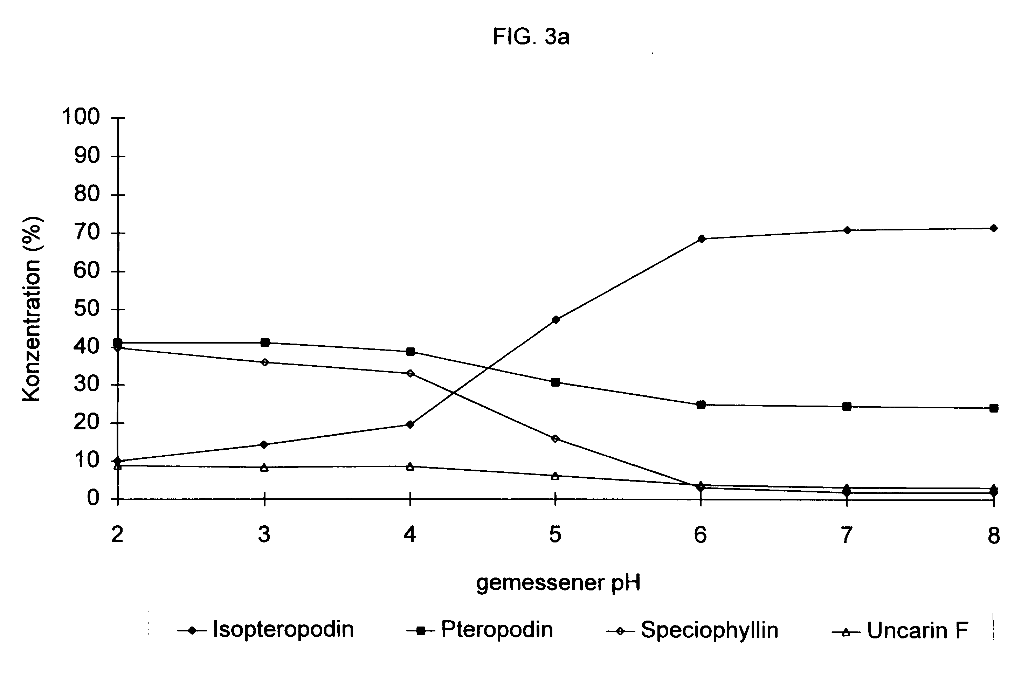

Es wurde festgestellt, dass die Isomerisierung bis zum Erreichen chemischer Gleichgewichte abläuft, die für dieses Lösungsmittel im sauren Bereich für den pH-Wert charakteristisch sind und im neutralen und basischen Bereich ein einziges Gleichgewicht bilden (Fig. 3a).

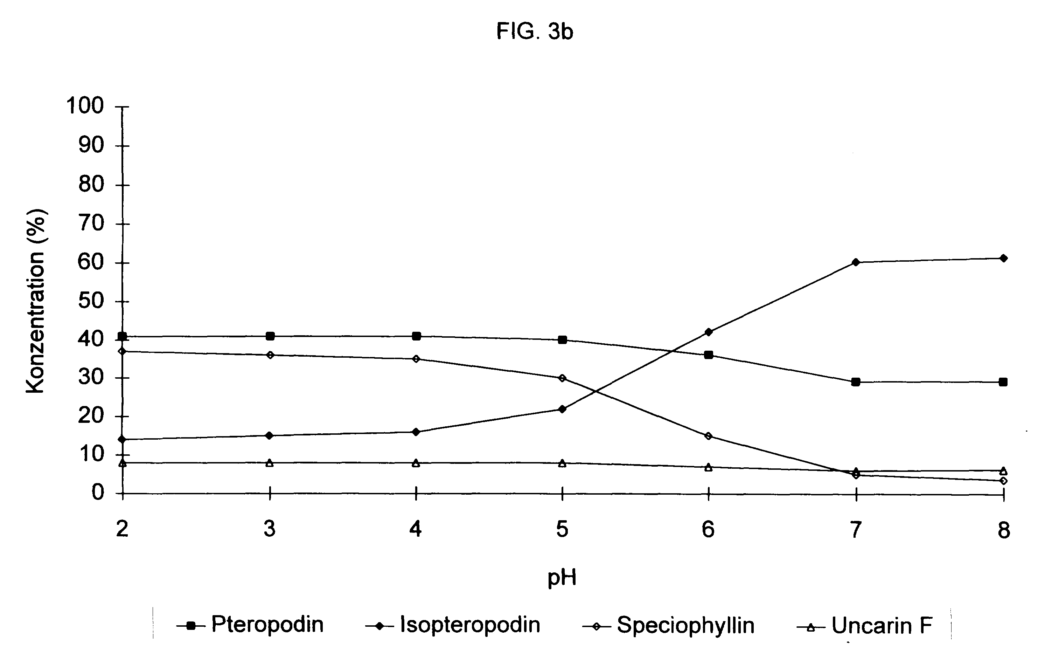

Beispielhaft wurde das pH-abhängige Isomerisierungsverhalten der Pteropodingruppe in Wasser untersucht (Fig. 3b).

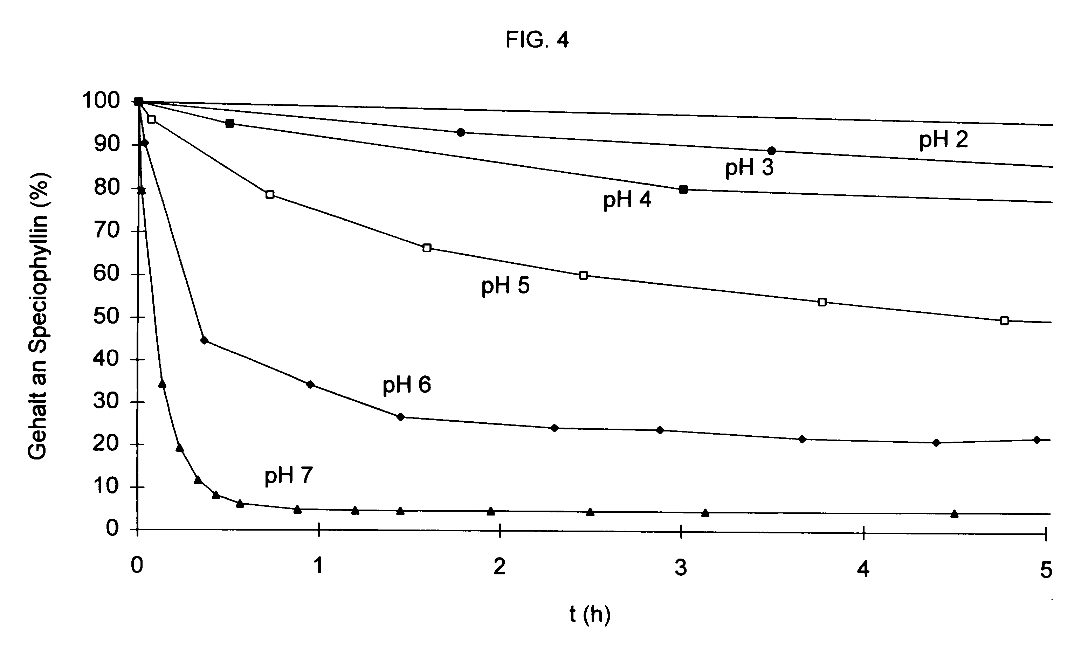

Des weiteren wurde festgestellt, dass die Reaktionssgeschwindigkeit mit steigender Protonenkonzentration abnimmt (Fig. 4).

Die Verwendung von Lösungsmitteln mit unterschiedlicher Polarität führte bei sonst gleichen Bedingungen zu unterschiedlichen chemischen Gleichgewichten. Auch die Temperatur hatte geringen Einfluss auf die chemischen Gleichgewichte (Tab. 4, 5).

**Tab. 4 :**

| *Einfluss von Temperatur und Lösungsmittel auf die Gleichgewichtsverteilung der Pteropodin-lsomeren (Temperaturbereich 25 - 50°C)* | | | |
|---|---|---|---|
| | | Lösungsmittel | |
| T (°C) | Alkaloid | Wasser | Methanol |
| 25 | Pteropodin | 29.6% | 22.9% |
| | Isopteropodin | 60.9% | 75.4% |
| | Speciophyllin | 4.3% | 0.4% |
| | Uncarin F | 5.2% | 1.3% |
| 37 | Pteropodin | 29.8% | 22.6% |
| | Isopteropodin | 59.6% | 75.5% |
| | Speciophyllin | 4.5% | 0.5% |
| | Uncarin F | 6.1% | 1.4% |
| 50 | Pteropodin | 29.6% | 23.5% |
| | Isopteropodin | 59.4% | 73.5% |
| | Speciophyllin | 4.6% | 1.0% |
| | Uncarin F | 6.4% | 2.0% |

**Tab. 5 :**

| *Einfluss von Temperatur und Lösungsmittel auf die Gleichgewichtverteilung der Mitraphyllin-lsomeren (Temperaturbereich 25 - 50°C)* | | | |
|---|---|---|---|
| T (°C) | Alkaloid | Lösungsmittel | |
| | | Wasser | Methanol |
| 25 | Mitraphyllin | 34.4% | 23.1% |
| | Isomitraphyllin | 65.6% | 76.9% |
| 37 | Mitraphyllin | 32.5% | 24.8% |
| | Isomitraphyllin | 67.5% | 75.2% |
| 50 | Mitraphyllin | 32.0% | 30.0% |
| | Isomitraphyllin | 68.0% | 70.0% |

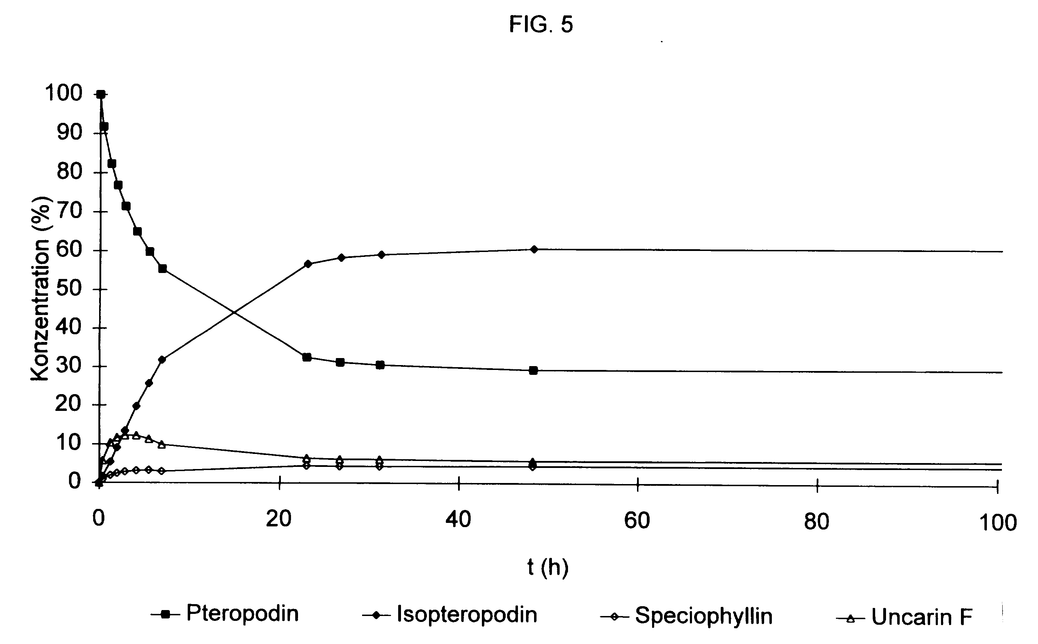

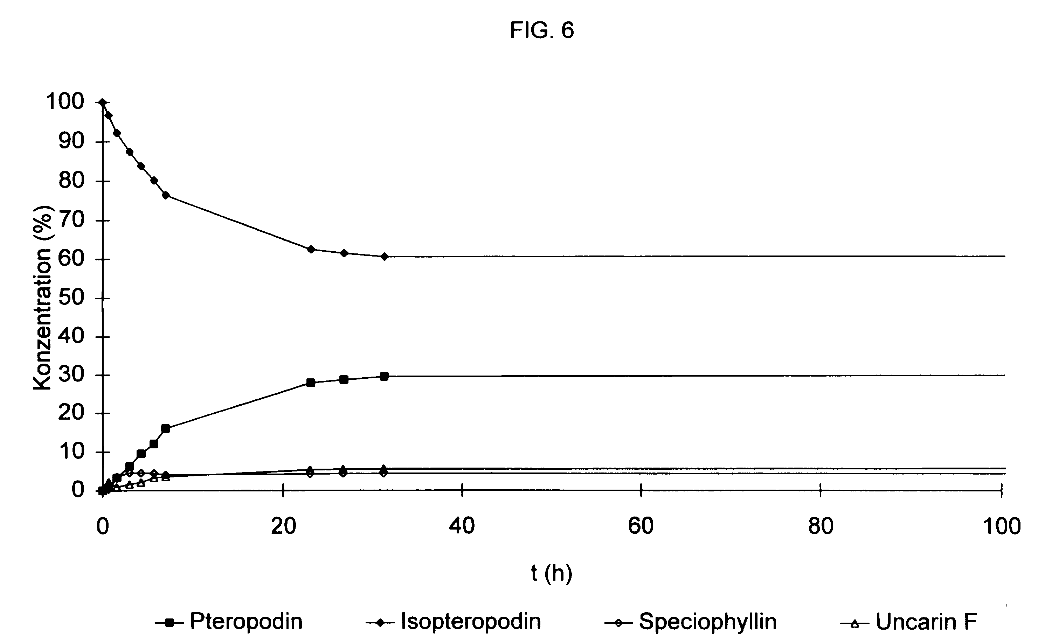

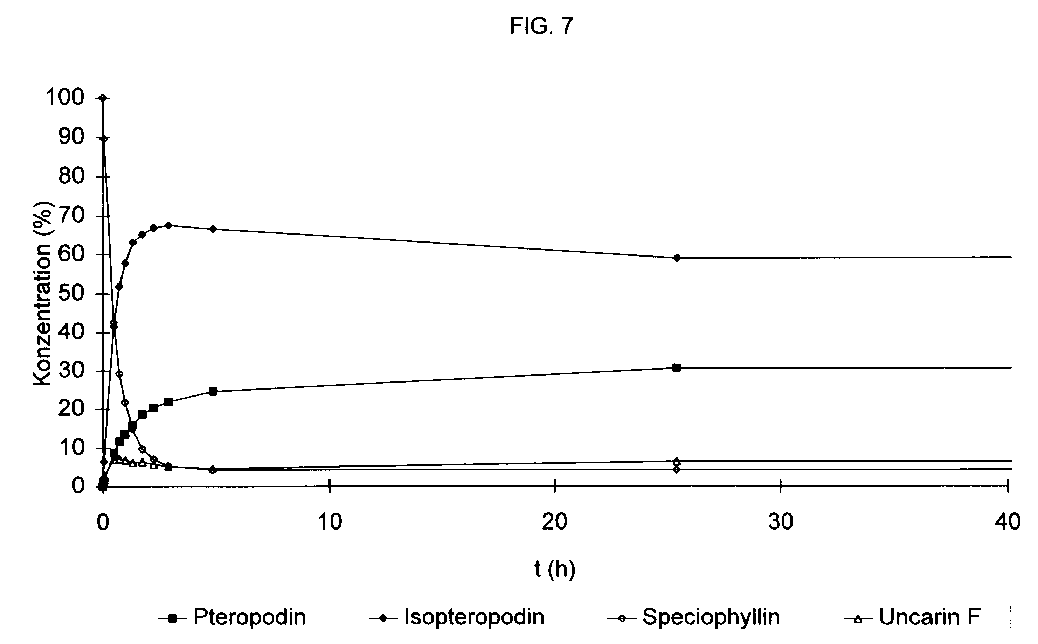

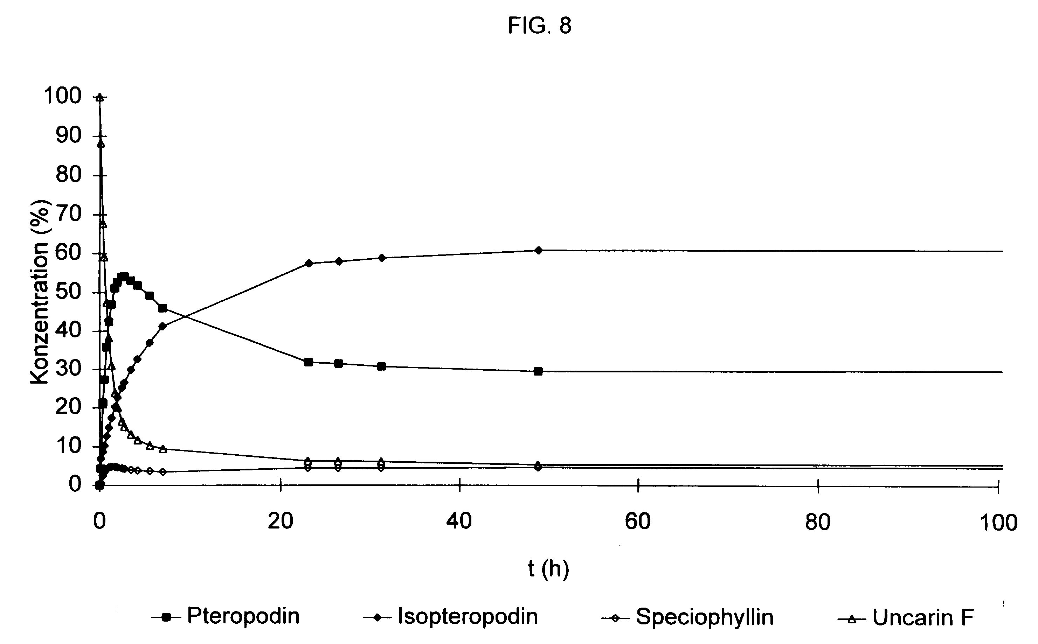

In Wasser wurden bei pH 7 die vier der gleichen Isomerenfamilie angehörenden Oxindolalkaloide Pteropodin, Isopteropodin, Speciophyllin und Uncarin F in einer Konzentration von je 18 mg/l gelöst und bei 37° C über 100 Stunden umisomerisiert. Während dieser Zeit wurden laufend Proben genommen und auf die Zusammensetzung der Isomeren untersucht (Fig. 5, 6, 7, 8).

Es zeigte sich, dass sich unter gleichen Bedingungen die einzelnen Isomeren unterschiedlich schnell umwandeln.

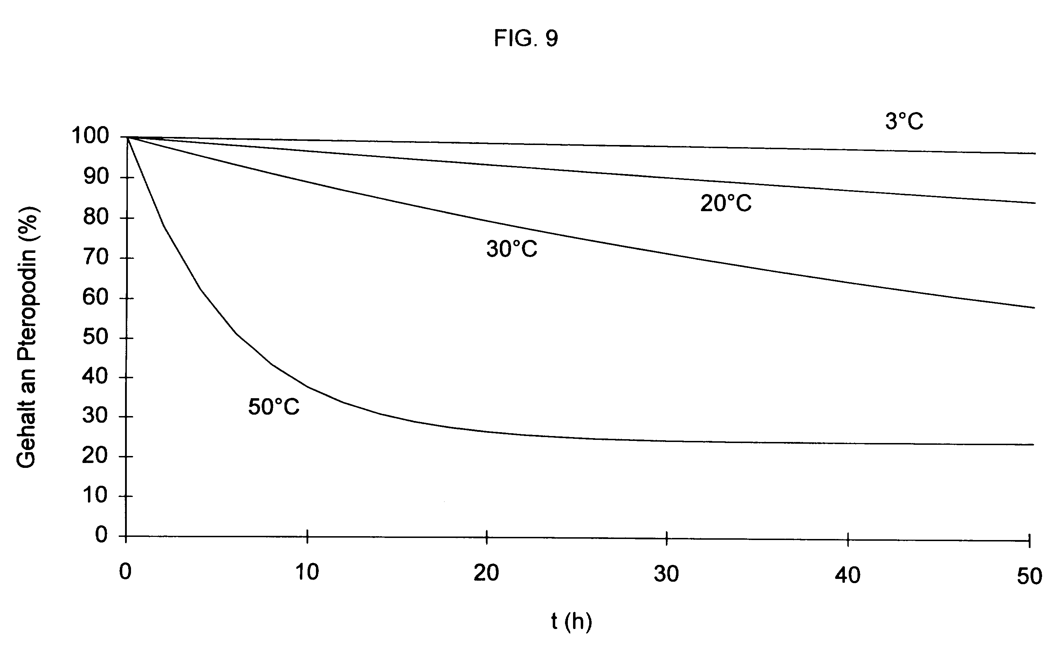

Untersucht wurde auch der Einfluss der Temperatur auf die Reaktionsgeschwindigkeit. Pteropodin wurde im zuvor beschriebenen Lösungsmittel in einer Konzentration von 20 mg/l gelöst und über 50 Stunden bei verschiedenen Temperaturen umisomerisiert. Wiederum wurden laufend Proben entnommen und auf ihren Pteropodingehalt geprüft (Fig. 9).

Eine Temperaturerhöhung der Lösung um 12° beschleunigt diese Reaktion auf das Fünffache.

### 3. Die Erfindung :

Die Herstellung definierter Isomerengemische aus Verbindungen mit spirozyklischen β- Aminocarboxyl- und / oder β-Aminocarbonyl-Systemen ist im technischen Massstab unter den bisher aus der Literatur bekannten Bedingungen nicht möglich. Auf das Verhältnis der Mengen von Produkt und Produktionsmittel ist ebensowenig Bedacht genommen worden wie auf eine befriedigende Produktionszeit. Auch sind die Angaben über die Isomerisierungsbedingungen mangelhaft.

Eigene Untersuchungen haben gezeigt, dass nur ausgewählte Lösungen eine technisch sinnvolle Isomerisierung ermöglichen. Solche Lösungen müssen folgende Eigenschaften in sich vereinen :
1. Sie müssen eine relativ hohe Dielektrizitätskonstante ε haben.
2. Sie müssen Wasserstoffbrücken als Protonendonoren ausbilden.
3. Sie müssen für die erfindungsgemässen Verbindungen gute Lösungseigenschaften haben.
4. Keiner ihrer Bestandteile sollte eine Basizität höher als die Basizität der zu isomerisierenden Substanzen haben.
5. Keiner ihrer Bestandteile sollte den Siedepunkt der Mischung so weit erniedrigen, dass entsprechende Energiezufuhr zur Beschleunigung der Isomerisierung nicht möglich ist.

Reine Lösungsmittel, die alle diese Eigenschaften in sich vereinigen, sind nicht bekannt. Erfindungsgemäss werden daher Lösungsmittelmischungen vorgeschlagen, deren gezielte Zusammensetzung am Beginn einer Isomerisierung und / oder deren In-Prozess-Veränderung und/oder deren Temperaturveränderung die Herstellung definierter Isomerengemische kennzeichnet.

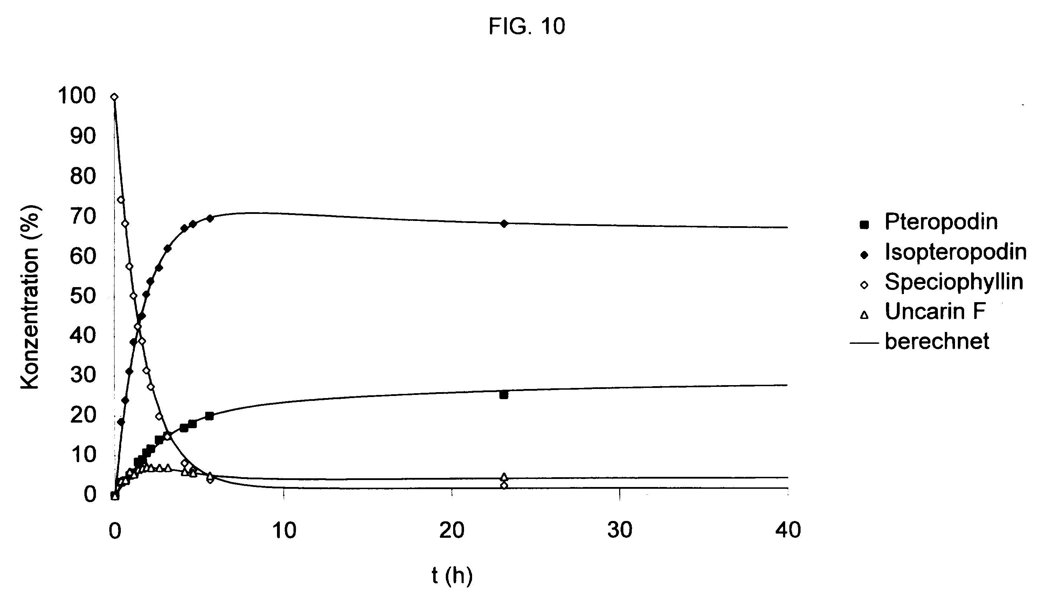

Um eine gezielte Anwendung dieses Verfahrens zu ermöglichen, wurde ein Rechenprogramm erstellt. Mit seiner Hilfe ist es möglich, für Lösungsmittel bei bestimmter Temperatur die sich bildenden Isomerengemische in ihrer zeitlichen Abfolge zu errechnen und den Zeitpunkt des Abbrechens der Reaktion im voraus zu bestimmen (Fig. 10).

### 4. Legende :

- Fig.1 :: Löslichkeit von Pteropodin in Methanol-Wasser-Gemischen bei 19°C
- Fig.2 :: Möglicher Mechanismus der Isomerisierung am Spiro-Zentrum
- Fig.3a:: Abhängigkeit der Isomerenverteilung im Gleichgewicht vom gemessenen pH-Wert in Wasser-Methanol (9:1)
- Fig.3b:: : Abhängigkeit der Isomerenverteilung im Gleichgewicht vom pH-Wert in Wasser
- Fig.4 :: Einfluss des pH-Wertes auf die Isomerisierungsgeschwindigkeit von Speciophyllin in Wasser bei 50°C
- Fig.5 :: Verlauf der Isomerisierung von Pteropodin in Wasser bei 37°C (gemessene Werte)
- Fig.6 :: Verlauf der Isomerisierung von Isopteropodin in Wasser bei 37°C (gemessene Werte)
- Fig.7 :: Verlauf der Isomerisierung von Speciophyllin in Wasser bei 37°C (gemessene Werte)
- Fig.8 :: Verlauf der Isomerisierung von Uncarin F in Wasser bei 37°C (gemessene Werte)
- Fig.9 :: Temperaturabhängigkeit der Isomerisierung von Pteropodin in Wasser-Methanol (7:3)
- Fig.10:: : Vergleich des gemessenen und berechneten Isomerisierungsverlaufes von Speciophyllin in Wasser bei 37°C

### 5. Bevorzugte Ausführungsbeispiele :

Ein bevorzugtes Ausführungsbeispiel gibt die Gewinnung von Pteropodin aus einem beliebigen Isomerengemisch der Pteropodin-Gruppe an. Das Ausgangsgemisch wird im Wasser bei pH 2 über 48 Stunden rückflussgekocht, dann wird die Lösung rasch, zumindest auf Raumtemperatur, abgekühlt und neutralisiert. Die Alkaloide fallen aus. Jetzt werden die Alkaloide durch Ausschütteln mit Diethylether extrahiert. Diese neue Lösung wird mittels Magnesiumsulfat getrocknet, dann wird das Lösungsmittel abgedampft. Um ein Isomerengemisch zu erhalten, aus dem sich Pteropodin durch Kristallisation gewinnen lässt, wird das aus der ersten Isomerisierungsstufe gewonnenen Gemisch in einem Lösungsmittel Methanol-Wasser im Verhältnis 2:8 gelöst und bei 37° C über drei Stunden umisomerisiert. Durch rasches Abkühlen wird dann die Isomerisierung unterbrochen. Wiederum werden die Alkaloide durch Ausschütteln mit Diethylether extrahiert. Auch diese Lösung wird mittels Magnesiumsulfat getrocknet, worauf das Lösungsmittel durch Abdampfen entfernt wird. Die Zusammensetzung der Alkaloide ist jetzt: Pteropodin 46 %, Isopteropodin 37 %, Speciophyllin 11 % und Uncarin F 6 %. Diese Isomerenmischung wird unter Erwärmen in t-Butylmethyl-Ether gelöst und dann langsam auf 18° C abgekühlt. Pteropodin kristallisiert aus und kann in sehr hoher Reinheit abfiltriert werden. Die im t-Butylmethyl-Ether verbliebenen Alkaloide können aus diesem gewonnen und dem gleichen Verfahren erneut zugeführt werden.

Ein anderes Ausführungsbeispiel zeigt die Gewinnung von Isopteropodin. Dazu wird ein beliebiges Gemisch von Isomeren der Pteropodin-Gruppe in einem Methanol-Wasser Gemisch (1:1) bei pH 7 gelöst und die Lösung über 60 Stunden bei 50° C gehalten. Dieser Lösung wird Essigsäure zur Konzentration von 0.2 mol/l zugegeben. Anschliessend wird das Methanol im Vacuum abdestiliert, wodurch die Lösung abkühlt. Isopteropodin fällt dabei aus.

Eine andere Methode zur bevorzugten Gewinnung von Isopteropodin geht vom Speziophyllin aus, das in Methanol gelöst wird, wobei nach 50 Stunden bei 50° eine Anreichung der Isopteropodin von über 70 % erreicht wird. Anschliessend wird das Methanol abdestilliert, der Rückstand wird in Essigsäure (Konzentration 0.2 mol/l) aufgenommen. Das Isopteropodin bleibt als Festsubstanz erhalten und wird abfiltriert.

Die beiden anderen Isomeren der Pteropodin-Gruppe werden gewonnen, indem ein beliebiges Ausgangsgemisch im Wasser bei pH 2 48 Stunden lang rückflussgekocht wird, worauf die Lösung rasch auf zumindest Raumtemperatur abgekühlt wird. Dann wird die Lösung neutralisiert. Die Alkaloide werden anschliessend mit Diethylether extrahiert. Die so gewonnene Lösung wird mit Magnesiumsulfat getrocknet, der Diethylether wird abgedampft.

Zur Gewinnung von Speciophyllin wird die pulverige Isomerenmischung in Essigester-Methanol im Verhältnis 9:1 gelöst und durch Säulenchromatographie getrennt. Als stationäre Phase wird Kieselgel verwendet, als Laufmittel das zuvor angegebene Lösungsmittel.

Uncarin F wird gewonnen, indem die aus dem ersten Produktionsschritt gewonnene Isomerenmischung unter Erwärmen in t-Butylmethyl-Ether gelöst und dann langsam abgekühlt wird. Pteropodin und Speciophyllin fallen aus und werden durch Filtration entfernt und können dem gleichen Verfahren erneut zugeführt werden. Das angereichert in der Lösung befindliche Uncarin F wird durch Abdampfen des Lösungsmittels und anschliessende chromatographische Reinigung gewonnen.

Die beiden Isomeren der Mitraphyllin-Gruppe können gewonnen werden, indem zur bevorzugten Gewinnung von Mitraphyllin ein Gemisch der beiden Isomeren in Wasser bei pH 2, zur bevorzugten Gewinnung von Isomitraphyllin in Methanol-Wasser gelöst wird. Die Lösung wird anschliessend über 10 Stunden auf 50° C gehalten und dann abgekühlt. Das im Fall der Isomitraphyllin-Gewinnung vorhandene Methanol wird abdestilliert. Anschliessend werden die Alkaloide mit Diethylether extrahiert. Wenn die so gewonnene Lösung eingeengt wird, kristallisiert Mitraphyllin aus und wird abfiltriert. Durch Abdampfen des restlichen Lösungsmittels wird Isomitraphyllin gewonnen.

## Patentansprüche

1. Verfahren zur Herstellung einer Lösung von isomeren, spirozyklischen Oxindolalkaloiden, wobei zumindest eines der isomeren Alkaloide in einem Lösungsmittelgemisch aus Wasser und einer mit Wasser mischbaren Substanz gelöst wird, dadurch gekennzeichnet, daß zur Herstellung einer Lösung mit einem definierten Isomerengemisch die Isomerisierung durch Wahl der dem Wasser zugemischten Substanz und durch Wahl der Konzentration der zugemischten Substanz im Lösungsmittelgemisch gesteuert wird, das eine Dielektrizitätskonstante von zumindest ε = 20 sowie die Fähigkeit aufweist, als Protonendonoren Wasserstoffbrücken auszubilden.

2. Verfahren nach Anspruch 1, dadurch gekennzeichnet, daß die Isomerisierungsgeschwindigkeit durch Wahl des pH-Wertes des Lösungsmittelgemisches gesteuert wird.

3. Verfahren nach Anspruch 1, dadurch gekennzeichnet, daß die Isomerisierungsgeschwindigkeit durch Wahl der Temperatur des Lösungsmittelgemisches gesteuert wird.

4. Verfahren nach einem der Ansprüche 1 bis 3, dadurch gekennzeichnet, daß die Isomerisierung bei Erreichen des gewünschten Isomerengemisches abgebrochen wird.

5. Verfahren nach den Ansprüchen 1 bis 4, dadurch gekennzeichnet, daß die Isomerisierung durch Trennung der gelösten Alkaloide vom Lösungsmittelsgemisch unterbrochen wird.

6. Verfahren nach den Ansprüchen 1 bis 5, dadurch gekennzeichnet, daß die Isomerisierung durch Quaternisierung, bevorzugt Protonierung, des Aminostickstoffes unterbunden wird.

7. Verfahren zur Gewinnung zumindest eines Oxindolalkaloides aus einer Lösung isomerer Alkaloide nach einem der Ansprüche 1 bis 6, dadurch gekennzeichnet, daß das Lösungsmittelgemisch entfernt und das Isomerengemisch in einem Lösungsmittel wiedergelöst wird, das bei Errichtung der Temperatur und/oder Änderung der Zusammensetzung durch Ausfällung die Alkaloide trennt.

## Claims

1. A process for the production of a solution of isomeric, spirocyclic oxindole alkaloids, wherein at least one of the isomeric alkaloids is dissolved in a solvent mix of water and a water-miscible substance, characterised in that to produce a solution with a defined isomer mix isomerisation is controlled by choice of the substance added to the water and by choice of the concentration of the added substance in the solvent mix, which has a relative permittivity of at least ε = 20 and has the capability of forming hydrogen bridges as proton donors.

2. A process according to claim 1 characterised in that the isomerisation rate is controlled by the choice of the pH-value of the solvent mix.

3. A process according to claim 1 characterised in that the isomerisation rate is controlled by the choice of the temperature of the solvent mix.

4. A process according to one of claims 1 to 3 characterised in that isomerisation is broken off when the desired isomer mix is reached.

5. A process according to claims 1 to 4 characterised in that isomerisation is interrupted by separation of the dissolved alkaloids from the solvent mix.

6. A process according to claims 1 to 5 characterised in that isomerisation is prevented by quaternisation, preferably protonisation, of the amino nitrogen.

7. A process for obtaining at least one oxindole alkaloid from a solution of isomeric alkaloids according to one of claims 1 to 6 characterised in that the solvent mix is removed and the isomer mix is redissolved in a solvent which upon establishment of the temperature and/or a change in the composition, by precipitation, separates the alkaloids.

## Revendications

1. Procédé pour la fabrication d'une solution d'alcaloïdes d'indoxyle spirocycliques isomères, dans lequel au moins l'un des alcaloïdes isomères est dissous dans un mélange de solvants composé d'eau et d'une substance miscible avec l'eau, caractérisé en ce que, pour fabriquer une solution ayant un mélange d'isomères défini, on commande l'isomérisation en choisissant la substance mélangée à l'eau et en choisissant la concentration de la substance mélangée dans le mélange de solvants qui a une constante diélectrique d'au moins ε = 20 ainsi que la capacité de former en tant que donneurs de protons des ponts hydrogène.

2. Procédé selon la revendication 1, caractérisé en ce qu'on commande la vitesse d'isomérisation en choisissant la valeur pH du mélange de solvants.

3. Procédé selon la revendication 1, caractérisé en ce qu'on commande la vitesse d'isomérisation en choisissant la température du mélange de solvants.

4. Procédé selon l'une des revendications 1 à 3, caractérisé en ce qu'on interrompt l'isomérisation lorsqu'on atteint le mélange d'isomères souhaité.

5. Procédé selon l'une des revendications 1 à 4, caractérisé en ce qu'on interrompt l'isomérisation par séparation des alcaloïdes dissous du mélange de solvants.

6. Procédé selon l'une des revendications 1 à 5, caractérisé en ce qu'on bloque l'isomérisation par quaternisation, de préférence par protonation, de l'azote aminé.

7. Procédé pour l'obtention d'au moins un alcaloïde d'indoxyle à partir d'une solution d'alcaloïdes isomères selon l'une des revendications 1 à 6, caractérisé en ce qu'on élimine le mélange de solvants et on redissout le mélange d'isomères dans un solvant qui sépare les alcaloïdes par précipitation par augmentation de la température et/ou changement de la composition.
